# EUROPEAN PATENT APPLICATION

(11) **EP 2 915 511 A1**
(43) Date of publication of application: **09.09.2015**
(21) Application number: 15160564.9
(22) Date of filing: 20.12.2013
(51) Int. Cl.: A61F 6/00

(54) **PACKING FOR CONDOM (VARIANTS)**

(30) Priority: 25.12.2012 RU 2012158208
(62) Divisional of application: 13824151.8
(71) Applicant: Meadowgrove Properties L.P., Edinburg EH7 5JA (GB)
(72) Inventor: Lepilin, Maxim, Evgenievich, 194354 St. Petersburg (RU)
(74) Representative: Andrae | Westendorp Patentanwälte Partnerschaft

(57) **Abstract**

The subject matter relates to device of packing for barrier contraceptives (condoms).

The packing for a condom consists of the covering 1 with the folded condom 2 inside. The covering 1 is connected to two side grippers 3, wherein each side gripper 3 with its one end is placed inside the covering 1 to be connected to, at least, one hook-shaped holder 4 located inside the covering 1 that is designed to fix the condom 2 within the covering 1. The covering 1 comprises the opening line 5 that is located by the central crosswise perimeter of the covering 1, that, when opening the covering 1, splits the covering 1 in two parts. The surface of the covering 1 is provided with at least two enforcement ribs 6 located parallel and symmetrical about the opening line 5 that function as pressure concentrators while opening the covering 1 by breaking it up. Each side gripper 3 surface may be ridged 8 by ruts to increase adhesion thus reducing the probability of sliding the fingers off when opening the packing.

## Description

### TECHNICAL FIELD

The subject application relates to device of packing for barrier contraceptives (condoms)

### BACKGROUND

Traditional packing for a condom represents a reliable covering made of flexible sheet material designed to store a condom sealed. For convenient usage such packings are provided with means to open the covering, for example a groove, a site with lower reliability on the edge of the packing, or an opening tape with one end left out to break the covering.

A deficiency of the traditional packing for a condom is a long and inconvenient packing opening process, because before using the condom one has to waste time looking for a covering opening tool provided with the packing, for example, the groove, and next, having taken the packing edges on both sides with both hands, pulling it apart to make a tear. Under the circumstances when a condom is about to be used, extra seconds wasted to make the abovementioned motions, may be critical for a person. Herein, there is a chance to damage a condom in case of negligent and hurried opening of the packing, because a person in tense emotional state may apply unnecessary extra force to tear the covering. Besides, when using a condom as designed, the contact between fingers and the condom itself is inevitable, thus compromising the hygiene required. The abovementioned deficiencies of the traditional packing for a condom may result in refusal to use the condom.

A packing for a condom is known (Patent Application WO9846495, published on October 22nd 1998) that comprises a covering wherein a condom is placed, the covering with a tear line at the center and side grippers attached to the covering edges designed to rest hand fingers when opening the covering. When using the packing known the side grippers are to be pulled apart in one plane. Herein, the covering is torn apart by the tear line to be split into two parts with a condom remaining in one part of the packing.

A deficiency of the known packing for a condom is the inevitable contact between hand fingers and the condom during its usage, because after opening the packing the condom must be extracted by hands. This compromises the hygiene required.

A packing for a condom is known (Patent EP1377242B1, published on May 24th 2006). This packing consists of a covering with a condom inside, and two flat side grippers connected to it and to each other. The covering comprises an opening line, located by its central crosswise perimeter, parallel to side grippers, that, when opened, splits the covering into two similar parts. Herein, the covering is provided with four hook-shaped holders located at the corners of the covering to fix the condom within. The upper surface of each side gripper is ridged.

The known packing is opened via pushing the side grippers with fingers resulting in tearing the packing apart by the opening line. Then the grippers are pulled apart to split the packing into two similar parts comprising the condom. Next, via catching the side grippers with fingers, the condom is put on the erect penis and removed from the packing via detaching it from the hook-shaped holders.

A deficiency of this packing for a condom is a long opening process resulting from reduced adhesion between the fingers and the side grippers surfaces held. This results from hands fingers sliding on the side grippers surfaces held due to moisture that forms before sexual intercourse because of hand fingers sweating. Besides, the existing connection of side grippers to each other necessitates additional effort to open the packing, as this connection of side grippers to each other has to be broken up, too.

### DISCLOSURE OF THE INVENTION

The object of the invention presented herein is to create a new packing for a condom that provides fast and convenient condom extraction simultaneously keeping the condom hygiene.

In one embodiment of the main technical solution the task set is solved in such a way, that a packing for a condom consisting of a covering with the condom within, two side grippers connected hereto and located inside the covering to fix the condom within the covering, wherein the covering has an opening line located by its central crosswise perimeter that, when opened, splits the covering into two parts, according to the subject matter, the covering surface is provided with at least two enforcement ribs located parallel to the opening line and symmetrical about it to act as pressure concentrators during opening the covering via breaking it up, wherein each side gripper with its one end is put inside the covering to be connected with at least one hook-shaped holder, while at the opposite side that is free of the covering, each side gripper is provided with a rest that fixes fingers position.

Embodiments for the technical solution presented are possible, wherein, upon the covering surface between the enforcement ribs, a place for possible themed image may be located, thus the opening line may be perforated, either straight or zigzagging, with side grippers surfaces that may be ridged.

Such a technical solution provides the following technical result, namely: to provide fast and convenient extraction of a condom simultaneously retaining its hygiene by means of:
- improved adhesion between the fingers and the packing while opening, due to side grippers provision with rests for fingers;
- reduced efforts needed to open the packing by means of the covering provided with enforcement ribs and by means of reduction, in comparison to the prototype, in a number of connecting elements within the packing;
- fingers not contacting the condom itself when extracting.

Enforcement ribs act as pressure concentrators when opening the covering via breaking it up, and their location along the opening line allows breaking up the covering in two parts with less efforts than needed by the known packings for condoms where such ribs are not presented and the covering is to be opened by tearing it apart by the opening line.

Optional embodiment with the opening line with zigzagging shape also provides for reduction of efforts, as the area of the opening zone would be enlarged due to a larger perforation area in comparison to the one with the straight opening line.

In another embodiment of the main technical solution, the packing for condoms consists of a covering with a condom within, the covering is attached to the two side grippers and hook-shaped holders located within the covering to fix the condom within the covering, wherein the covering has an opening line located at the central crosswise perimeter thereof, the opening line, when opening the covering, splits the covering in two parts, according to the subject matter, the covering surface is provided with at least two enforcement ribs located parallel to the opening line and symmetrical about it that function as pressure concentrators when opening the covering by breaking it up, wherein each side gripper is placed with one end inside the covering to be connected to, at least, one hook-shaped holder, and wherein the covering, together with side grippers, in cross section view has an ellipse shape, while in axial section view it has convexo-concave profile that narrows towards the side grippers edges.

Embodiments for the technical solution presented are possible, wherein, upon the covering surface between the enforcement ribs, a place for possible themed image may be located, thus the opening line may be perforated, either straight or zigzagging, with side grippers surfaces that may be ridged.

Such a technical solution provides the following technical result, namely: to provide fast and convenient extraction of a condom simultaneously retaining its hygiene by means of:
- reduced efforts needed to open the packing by means of the covering provided with enforcement ribs, making the packing of convexo-concave shape and by means of reduction, in comparison to the prototype, in a number of connecting elements within the packing;
- fingers not contacting the condom itself when extracting.

Enforcement ribs act as pressure concentrators when opening the covering via breaking it up, and their location along the opening line allows breaking up the covering in two parts with less efforts than needed by the known packings for condoms where such ribs are not presented and the covering is to be opened by tearing it apart by the opening line.

### BRIEF DESCRIPTION OF THE DRAWINGS

Examples of particular embodiments of the subject matter as well as the practical applicability thereof are disclosed herein with reference to the following description and the drawings.
Fig.1 shows lateral view of the packing for a condom (with transparent covering) of claim 1, wherein:
   1 represents the covering;
   2 represents the condom;
   3 represents a side gripper;
   4 represents a hook-shaped holder;
   5 represents an opening line;
   6 represents an enforcement rib;
   7 represent a finger rest;
   8 represents ridging.
Fig.2 shows the general view of the packing for a condom (straight opening line) according to claim 1.
Fig.3 shows the general view of the packing for a condom (zigzagged opening line) according to claim 1.
Fig.4 shows the top view for the opened packing for a condom (zigzagged opening line) according to claim 1.
Fig.5 shows the top view for the packing for a condom (zigzagged opening line) according to claim 2.
Fig.6 shows the lateral view for the packing for a condom (zigzagged opening line) according to claim 2.

### IMPLEMENTATION OF THE INVENTION

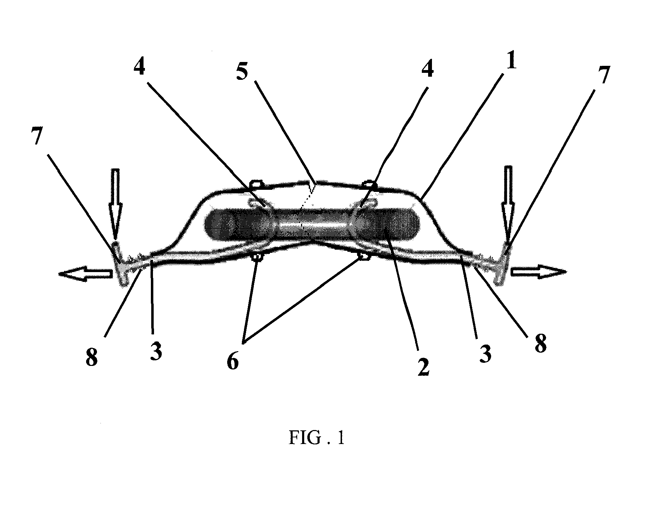

The packing for a condom according to claim 1 (Fig.1) consists of the covering 1 with the folded condom 2 inside. The covering 1 is connected to two side grippers 3, wherein each side gripper 3 with its one end is placed inside the covering 1 to be connected to, at least, one hook-shaped holder 4 located inside the covering 1 that is designed to fix the condom 2 within the covering 1.

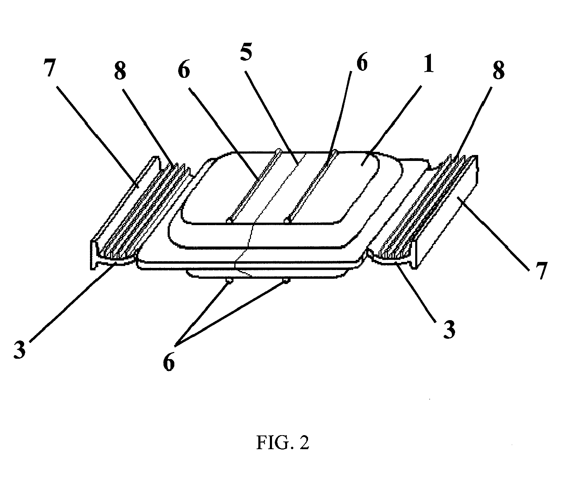

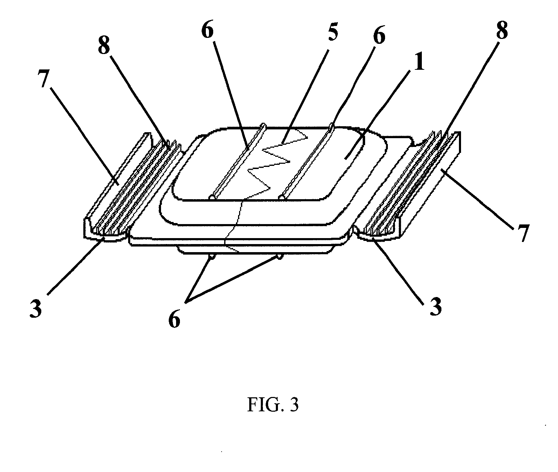

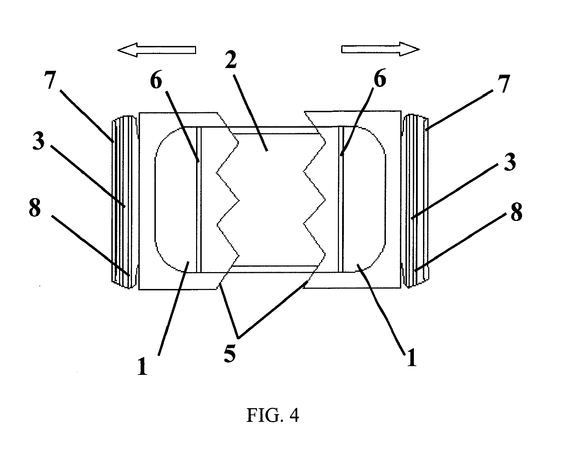

The covering 1 comprises the opening line 5 (Fig.2, 3) located by the central crosswise perimeter of the covering 1, that, when opening the covering 1, splits the covering 1 in two parts (Fig.4).

The surface of the covering 1 is provided with at least two enforcement ribs 6 located parallel and symmetrical about the opening line 5 that function as pressure concentrators while opening the covering 1 by breaking it up. A particular embodiment may have, for example, two, four or six enforcement ribs 6 on each side of the covering 1.

Each side gripper 3, on the opposite side that is free of the covering 1, is provided with the rest 7 (Fig.1, 2, 3, 4) to fix fingers location (not shown on drawing).

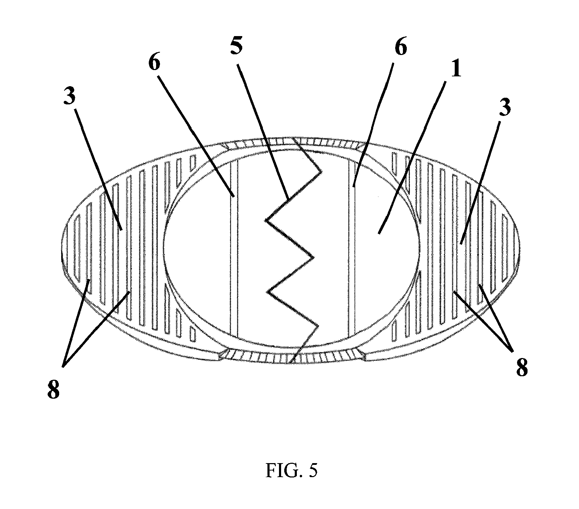

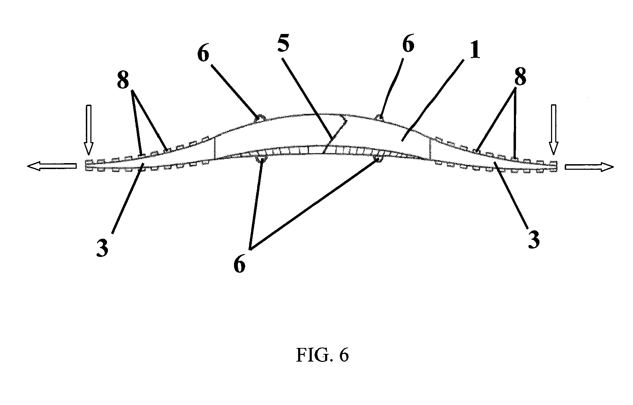

Main elements of the packing for a condom according to claim 2 (Fig.5, 6) correspond the abovementioned ones (Fig.1) with the exception of the rests 7 presented herewith. Besides, the covering 1, together with side grippers 3 in its cross section has a shape of an ellipse (displayed by drawing tentatively), while in its axial section it has the convexo-concave profile (displayed by drawing tentatively) that narrows gradually towards the side grippers 3 edges. Implementing the packing shape this particular way facilitates faster opening of the covering 1 resulting from less efforts needed to break it up by the opening line 5.

The surface of each side gripper 3 may have ridging 8 due to ruts (displayed by drawing tentatively) to increase adhesion thus reducing the probability of sliding the fingers off when opening the packing.

The surface in between the enforcement ribs 6 on both sides of the covering 1 is implemented to provide for applying a topical image thereon (not shown on drawing).

The covering 1, for example, may consist of two layers (an upper one and a lower one) of polymer material and comprise additional lubricant (not shown on drawing) to keep the condom undamaged and with good appearance. Herein the covering 1 is implemented by means of leak-proof gluing or soldering the two layers of the polymer material (displayed by drawing tentatively).

Enforcement ribs 6 may be implemented, for example, by means of pressing or superimposing or some other way.

The present invention is used as follows.

To use the condom 2 as intended the packing for a condom (Fig.4) is to be opened. To do so, one has to take the side grippers 3 with both hands and to pull the grippers up or down (in vertical plane) resulting in breaking the covering 1 by the opening line 5. Next, to fully split the covering 1 in two parts one has to pull the side grippers 3 apart (in horizontal plane). Herein, due to the condom 2 fixation by the hook-shaped holders 4 the side grippers 3 are provided with, the condom 2 would also be stretched and the condom 2 ring diameter would also increase. This provides an opportunity for putting the condom onto the erect penis (not shown on drawing) fast and convenient, without touching the condom 2 with fingers. Upon the complete unwinding of the condom 2 ring, the hook-shaped holders 4 can be easily detached from the condom 2.

The opened condom packing is to be disposed of.

Thus, the present invention provides for the following technical results: fast and convenient condom extraction, while simultaneously retaining its hygiene.

The present invention further relates to the following aspects.

According to a first aspect, a packing for a condom is disclosed, consisting of a covering with a condom inside, the covering is connected to two side grippers and hook-shaped holders located inside the covering and designed to fix the condom within the covering, wherein the covering includes an opening line that is located by its central crosswise perimeter to split, when opening, the covering in two parts, wherein the covering surface is provided with at least two enforcement ribs located parallel to the opening line and symmetrical about it to function as pressure concentrators during opening it by breaking it up, wherein each side gripper is, with one end, placed inside the covering to be connected with, at least, one hook-shaped holder, and, on the opposite side that is free of the covering, with a finger rest provided that fixes fingers position.

In one embodiment of the aforementioned first aspect, the surface between the enforcement ribs comprises a site for optional topical image application.

In another embodiment of the aforementioned first aspect, the opening line is made straight.

In another embodiment of the aforementioned first aspect, the opening line is made zigzagging.

In another embodiment of the aforementioned first aspect, the opening line is made perforated.

In another embodiment of the aforementioned first aspect, the side grippers surface is ridged.

According to a second aspect, a packing for a condom is disclosed, consisting of a covering with a condom inside, the covering is connected to two side grippers and hook-shaped holders located inside the covering and designed to fix the condom within the covering, wherein the covering includes an opening line that is located by its central crosswise perimeter to split, when opening, the covering in two parts, wherein the covering surface is provided with at least two enforcement ribs located parallel to the opening line and symmetrical about it to function as pressure concentrators during opening it by breaking it up, wherein each side gripper is, with one end, placed inside the covering to be connected with, at least, one hook-shaped holder, wherein the covering, together with side grippers, in cross section view has an ellipse shape, while in axial section view it has convexo-concave profile that narrows towards the side grippers edges.

In one embodiment of the second aspect, the surface between the enforcement ribs comprises a site for optional topical image application.

In another embodiment of the second aspect, the opening line is made straight.

In another embodiment of the second aspect, the opening line is made zigzagging.

In another embodiment of the second aspect, the opening line is made perforated.

In another embodiment of the second aspect, the side grippers surface is ridged.

## Claims

1. Packing for a condom, consisting of a covering with a condom inside, the covering is connected to two side grippers and hook-shaped holders located inside the covering and designed to fix the condom within the covering, wherein the covering includes an opening line that is located by its central crosswise perimeter to split, when opening, the covering in two parts, wherein the covering surface is provided with at least two enforcement ribs located parallel to the opening line and symmetrical about it to function as pressure concentrators during opening it by breaking it up, wherein each side gripper is, with one end, placed inside the covering to be connected with, at least, one hook-shaped holder, wherein the covering, together with side grippers, in cross section view has an ellipse shape, while in axial section view it has convexo-concave profile that narrows towards the side grippers edges.

2. The packing of claim 1, wherein the surface between the enforcement ribs comprises a site for optional topical image application.

3. The packing of claim 1, wherein the opening line is made straight.

4. The packing of claim 1, wherein the opening line is made zigzagging.

5. The packing of claim 1, wherein the opening line is made perforated.

6. The packing of claim 1, wherein the side grippers surface is ridged.
